# EUROPEAN PATENT APPLICATION

(11) **EP 4 756 092 A1**
(43) Date of publication of application: **10.06.2026**
(21) Application number: 25220019.1
(22) Date of filing: 02.12.2025
(51) Int. Cl.: D04H 3/16, D04H 1/407, D04H 1/558, D04H 1/56, A61F 13/534, B32B 5/24, B32B 5/30

(54) **APPARATUS FOR MAKING NONWOVEN FABRIC**

(30) Priority: 04.12.2024 IT 202400027495
(71) Applicant: Minunni, Rosa Maria, 20095 Cusano Milanino (MI) (IT)
(72) Inventor: Minunni, Rosa Maria, 20095 Cusano Milanino (MI) (IT)
(74) Representative: Lunati & Mazzoni S.r.L.

(57) **Abstract**

An apparatus (100) is provided for making nonwoven fabric including at least one plant for making nonwoven fabric developing along a main axis (1a) and a main plane (1b) and configured to dispense via an outlet (10a) a plurality of either polymeric or cellulose or viscose filaments along and parallel to one or more dispensing directions (4a) transverse to the main plane (1b) forming from the outlet (10a) a layer of filaments along or parallel to a dispensing plane (100a) transverse to the main axis (1a) and the main plane (1a); and a conveying station (12) downstream of the outlet (10a) and including at least calendering means (13) spaced from the outlet (10a) by a collection distance of between 0 m and 5 m; wherein the conveying station (12) lacks a belt or mat collection surface placed upstream of the calendering means (13) in such a way that the layer is directly calendered into the calendering means (13) without being previously deposited on a the belt or mat collection surface.

## Description

The present invention relates to an apparatus for making nonwoven fabric of the type specified in the preamble to the first claim.

Specifically, the present invention relates to an apparatus including at least one plant comprising a spunbond spinneret, or the combination of spunbond and melt-blown spinnerets, either of the cusp or coaxial multi-row type, designed to enable the distribution of polymeric fluid exiting the plant in the form of extruded polymeric filaments to obtain nonwoven fabric.

In addition, the present invention also relates to a plant for enabling the combination of extruded polymer filaments with cellulose filaments to obtain nonwoven fabric.

In addition, the present invention relates to an article, within which are layered continuous polymeric filaments possibly combined with cellulose or other synthetic materials in fibrous or particulate form, and thr related process for the formation of such an article, which can be applied in filtration or, especially if the particles are liquid absorbents, in absorbent hygienic articles.

As is known, nonwoven fabric, or NWF, is an industrial product similar to a fabric but obtained by processes other than weaving and knitting. Therefore, within a nonwoven fabric, the fibers have a random pattern, with no identifiable ordered structure while in a fabric the fibers have two prevailing and orthogonal directions between them, usually called weft and warp.

Currently, a plurality of products containing NWF are manufactured depending on the manufacturing technique used, mainly connected to the use that is made of the product itself.

In particular, a distinction is made between high quality NWFs for sanitary products, and low quality NWFs used especially for geotex.

From a technical point of view, nonwoven fabrics can be basically divided into spunlace, spunbond and multirow coaxial or cusp melt-blown fabrics.

The spunlace fabric undergoes processing that gives the material equidirectional resistance. Thanks to this property, to the possibility of being produced in different materials such as viscose, polyester, cotton, polyamide and microfibre, to the two possible finishes, i.e. smooth or perforated, and to the multitude of smooth or printed colours, spunlace is suitable both for the sanitary sector and for the automotive, cosmetic, industrial or single-use sectors.

Spunbond, usually made of polypropylene, is a nonwoven fabric that finds multiple applications in the agricultural, sanitary, construction, furniture, mattress and other related sectors. By means of an appropriate treatment, it is possible to create a series of highly specific products for each sector: fluorescent, soft calendered, anti-mite, fireproof, antibacterial, antistatic, anti-UV and others. Numerous finishes can also be applied to the Spundbond, such as printed, laminated, flexographic printed laminate and self-adhesive.

The production plants of the spundbond nonwoven fabric essentially include at least one inlet conduit of the polymeric substance, a polymer extrusion head, a polymer distributor or breaker plate and a spinneret adapted to make the actual spundbond yarn that is deposited on a conveyor belt.

The mentioned elements are each arranged accordingly and adjacent to each other in such a way as to allow the processing of the polymer and the distribution of the NWF spundbond. More in detail, the polymer inside the dispensing conduit is pushed under pressure and at high temperatures, usually above 200°C, towards the extrusion head. In this regard, in general, a pressure control is carried out, for example by means of a pressure switch, to ensure the continuity of the output yarn and the precision of the deposition process.

The extrusion head distributes the polymer along a distribution surface through which the molten polymer reaches the distributor. Between the distributor, or breaker plate, and the extrusion head there is a filter consisting of a steel sheet with a thickness usually varying between 0.8 and 1.6 mm and including fine meshes, for example, with nominal dimensions comprised between 20 and 110 µm. Basically, therefore, the filter mentioned is a stretched net.

After passing inside the filter, the molten polymer enters the distributor that accompanies the polymer towards the spinneret where the polymer is extruded into filaments constituting the spundbond NWF. In detail, the filter has the purpose of blocking particles or polymer pigments, not perfectly dissolved or in any case larger, which by entering the spinneret could obstruct the extremely small NWF extrusion holes.

The NWF melt-blown is made through specific spinnerets in order to achieve higher technical characteristics than previous TNTs. In fact, the melt-blown fabric is characterised by fiber with high filtering power for both liquid and aeriform substances.

The melt-blown nonwoven fabric production plants consist of a box that encloses the melt-blown fiber manufacture device and all the parts that are necessary for the process to function at its best.

The known cusp melt-blown plants comprise an extrusion head, a cusp distributor, and an air blade.

The multi-row coaxial melt-blown plants provide for stretching the polymer that comes out of tubes, arranged in rows, through the air which, in a coaxial manner, passes from the outside of the tube and pushes the fiber downwards.

In particular, the multi-row coaxial melt-blown type plants comprise components defining coaxial holes, arranged in rows and adapted to house at least part of the aforementioned tubes transiting coaxially inside the holes in such a way as to allow the diffusion of polymeric fluid and, at the same time, to allow the diffusion of air or gas from at least part of the holes. Usually, these plants include devices, called spin packs, including a plurality of different components adapted to interact with each other. Usually, a spin pack consists of a spinneret and a diffusion device including one or more components called air plate.

In even greater detail, the currently known multi-row coaxial spunbond and/or melt-blown spunbond and/or melt-blown plant spinnerets include a plate including first holes suitable for accommodating tubing configured to distribute polymeric fluid, and second holes separate from the first holes and suitable for allowing air or gas passage, and further a jig, separate from or in one piece with the plate, including a plurality of third holes centered with respect to the first holes, in fluid passage connection with said second holes and suitable for housing part of the tubing and allowing, at the same time, the passage of said air or gas. The prior art described comprises some important drawbacks.

Specifically, especially spunbond technology usually involves that downstream of the spinneret, that is, in a position following the exit from the filament spinneret, there are nozzles designed to blow the filaments with jets of air for the purpose of cooling them. Cooling the filaments before deposition on a spool tape is critical because it prevents adjacent filaments from sticking together due to heat.

At the same time, however, the air jets cannot exert excessive pressure on the filaments as otherwise the filaments would risk being deflected from their exit trajectory, still leading to undesirable mutual attachment of the filaments.

Added to this is the fact that since the cooling nozzles are placed laterally to the filament exit trajectory, the reduced pressure with which the air blows the filaments means that the central filaments cannot be, as shielded by the laterals, cooled efficiently or at least similarly to the lateral filaments. Thus, the technical problem related to unintentional splicing of filaments, with loss of performance in terms of nonwoven fabric, remains.

In addition to the above, it is evident that in order to ensure proper cooling of the filaments, it is necessary to increase the mutual distance, i.e., the spacing, between the output filaments. This implies that currently known spinnerets cannot include a high density of holes or dispensing tubes with the consequence that high density fabric layers cannot be made.

Thus, the layers of nonwoven fabric made with the currently known spinnerets are not of particularly high-performance when placed under traction in different directions.

In fact, while the nonwoven fabrics thus made are very robust along the main development direction of the plant, they are not so perpendicular to that direction.

The consequence of this weakness is, for example, very impactful in the manufacture of diapers and wet wipes.

In fact, the above items are normally made by overlapping a plurality of layers to form the complete layered structure of the item itself.

Specifically, normally, a conventional diaper is made following a sequence (generally referred to as S, S, MB, MB, S) of layering in which there are two spunbond type sheets, two melt-blown type sheets, and an additional spunbond type sheet; the wipe has similarities although it also includes intermediate portions that may include particulates.

In any case, the items just described, as implemented, have some major drawbacks.

First, they are made by layering on the same tape, which implies a plurality of different steps and high lead times. Moreover, this aspect also implies more costs resulting not only from the way it is made, but also from the fact that a greater number of layers increases the cost of the item.

In addition, some layers are double due to the fact that, especially the conventional spunbond spinnerets previously described, have limitations about the density of filament delivery holes. Therefore, it is necessary to make the spunbond part in two layers, therefore in two separate stages and/or devices, typically a plurality of booths arranged in series each equipped with a containment chamber within which there is a spinneret of the type needed to make the desired layer.

In addition, and as already anticipated, it is also known that some items may include particulate materials. The latter are widely used within absorbent structures for absorbent items, for example for personal hygiene, such as disposable diapers for children, training pants for children or underwear for adult incontinence, which are designed to absorb and contain body exudates, especially urine, or in other moisture-retaining items such as wet wipes.

These absorbent articles comprise several layers which perform different functions, typically including a topsheet, a backsheet and, among other layers, an absorbent core. The absorbent core must be able to absorb and retain liquid exudates for a prolonged period of time, such as overnight for a diaper, should minimize re-wetting to keep the wearer dry, and avoid soiling clothing or sheets. Modern absorbent cores typically comprise absorbent structures composed of super-absorbent polymer (SAP) particles, also called absorbent gelling materials (AGM), and fibrous materials, which may be natural, such as cellulose fibres, modified natural, such as regenerated cellulose-based materials, or synthetic fibres, for example viscose.

It is well known that the absorbent structures can be formed "in-line" or "in-situ" on a converting line to form the complete absorbent article; see for example WO2022/120693A1, which discloses an absorbent core for use in an absorbent article, comprising a liquid-permeable top cover layer, a bottom cover layer and a high-fill-coefficient core layer between the top and bottom cover layers, and first and second super-absorbent polymers which at least partially penetrate into the high-fill-coefficient core layer, in addition to adhesively-fixed cover layers. Furthermore, WO2014/001487 discloses particles embedded in a porous fabric and ultrasonically immobilized between the cover layers.

However, these approaches require that every line for the production of articles, also known as a converter, be equipped with appropriate handling systems for adding particles, as well as unwinding and joining systems for the preformed tapes. Furthermore, the formation of the absorbent core may limit the overall production volume.

As an alternative to forming the core in-line, composite absorbent tapes comprising particles, such as super-absorbents, may be formed off-line at high production volumes, which may be delivered as so-called roll stocks to the conversion lines for forming articles to be packaged and/or combined with other elements to form the absorbent articles, thereby advantageously simplifying the conversion equipment and process and offering production cost advantages due to the high production volumes.

In general, although off-line forming of absorbent structures offers advantages over in-line forming, such as a very high production volume with a single production unit, there is still a need to improve the economics and/or properties of the resulting absorbent structures. Furthermore, the use of adhesives in the absorbent structure complicates recycling, in particular that of factory waste, and can have a negative impact on consumers' perception as an unnecessary chemical.

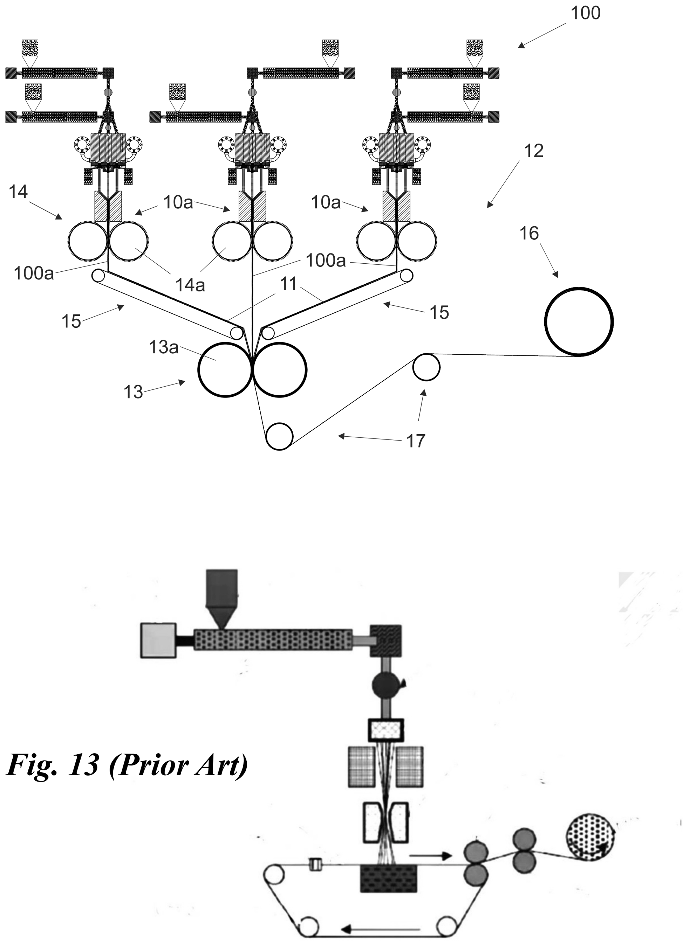

With reference to typical plants of the prior art, as shown for example in Fig. 13, an additional drawback inherent in the technology employed is that, normally, filaments are deposited on a deposition belt or mat on which, in the case of a plurality of plants placed in succession, a plurality of filament layers are layered.

This layering means, during calendering, that the layers and filaments are efficiently compressed and compacted, therefore, especially in the formation of multilayer items such as diapers, it results in an inherent fragility of the individual layers, and of the joint, which makes the item perform less well.

In this situation, the technical task underlying the present invention is to devise an apparatus for making nonwoven fabric that can substantially overcome at least part of the above-mentioned drawbacks.

Within the scope of said technical task, it is an important purpose of the invention to obtain a plant to make nonwoven fabric to increase the density of polymer filament delivery holes within the same plant, preferably of the spunbond type.

Therefore, another important purpose of the invention is to realize a plant for making nonwoven fabric that enables layers of nonwoven fabric, normally made with several distinct spinnerets, to be made with a single spinneret.

Thus, an important task of the invention is to realize an apparatus for making nonwoven fabric to reduce the cost of producing a layer for a sanitary item such as a diaper, wet wipe or other similar items.

In addition, a further purpose of the invention is to realize an apparatus for making nonwoven fabric that enables layered items to be made by reducing the process steps and time required to complete the item.

In particular, in this regard, a further task of the invention is to realize an apparatus for making nonwoven fabric that enables the production of high-performance layered items in which the layers are highly durable, difficult to wear out, and the joints between the different layers are performed efficiently.

Thus, a further task of the invention is to obtain an apparatus for making nonwoven fabric that can combine different technologies, e.g., spunbond and melt-blown, for obtaining the items.

In addition, a further task of the invention is to obtain an apparatus for making nonwoven fabric capable of enabling the combination of extruded polymer filaments with cellulose filaments to obtain nonwoven fabric.

Thus, a further purpose of the invention is to make an item, within which are layered continuous polymeric filaments possibly combined with cellulose or other synthetic materials in fibrous or particulate form, and related process for the formation of such an article, which can be applied in filtration or, especially if the particles are liquid absorbents, in absorbent hygienic articles.

In particular, an additional task of the invention is to reduce the complexity and cost of making the item.

The technical task and the specified purposes are achieved by an apparatus for making nonwoven fabric as claimed in the attached claim 1.

Preferred embodiment are highlighted in the dependent claims.

The features and advantages of the invention are clarified below by the detailed description of preferred embodiments of the invention, with reference to the attached drawings, in which:

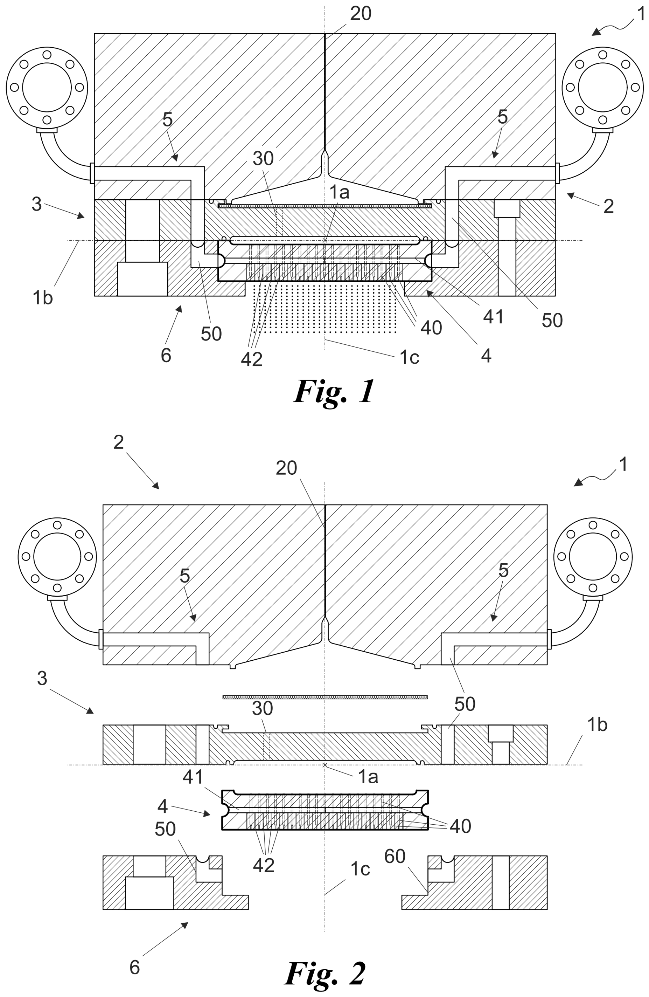

Fig. 1 shows a cross-sectional view of a plant to make nonwoven fabric according to the invention;
Fig. 2 illustrates an exploded view of the plant of Fig. 1;

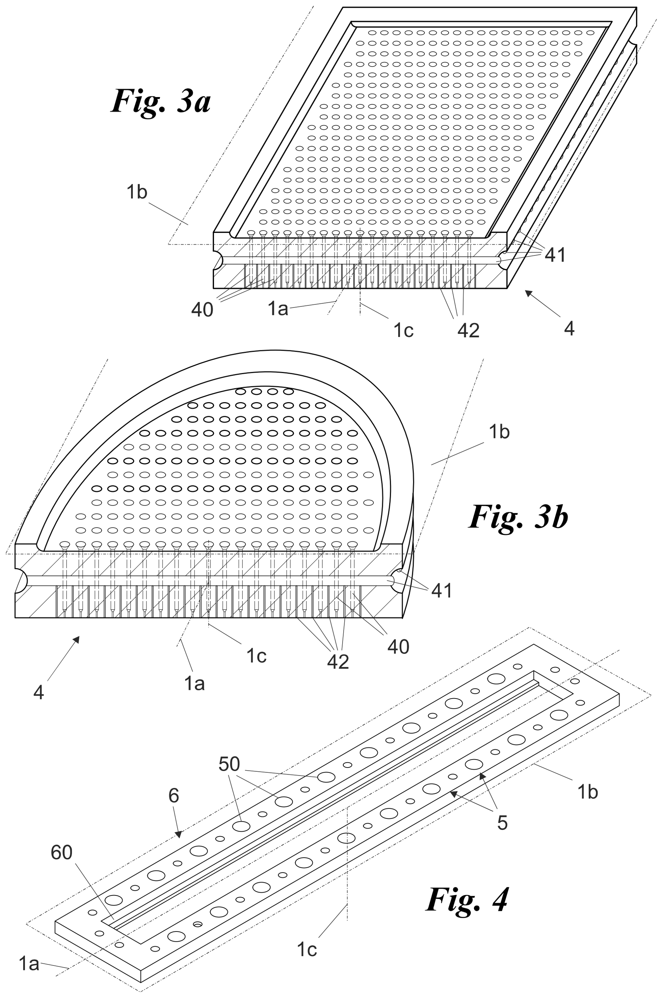

Fig. 3a is a cross-sectional perspective view of a rectangular spinneret of a plant to make nonwoven fabric according to the invention;
Fig. 3b is a cross-sectional perspective view of a circular spinneret of a plant to make nonwoven fabric according to the invention;
**Fig. 4** shows a perspective view of a spinneret holder of a plant to make nonwoven fabric according to the invention;

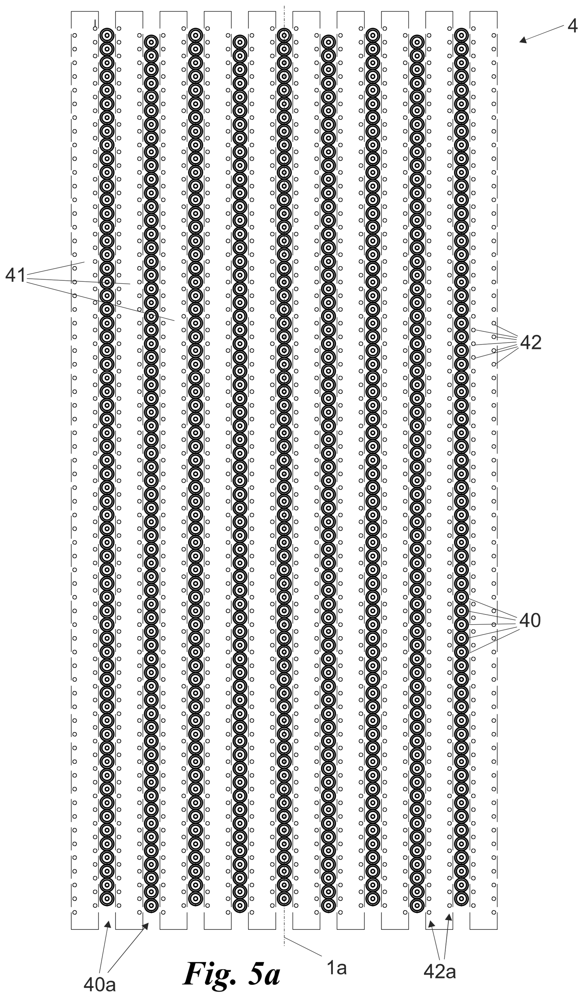

**Fig. 5a** illustrates an upper schematic view, i.e., from the point of view of the dispenser, of the first holes and slits and second holes, shown in hatches, of a spinneret of a plant to make nonwoven fabric according to the invention in which the slits are parallel to the main axis and continuous;

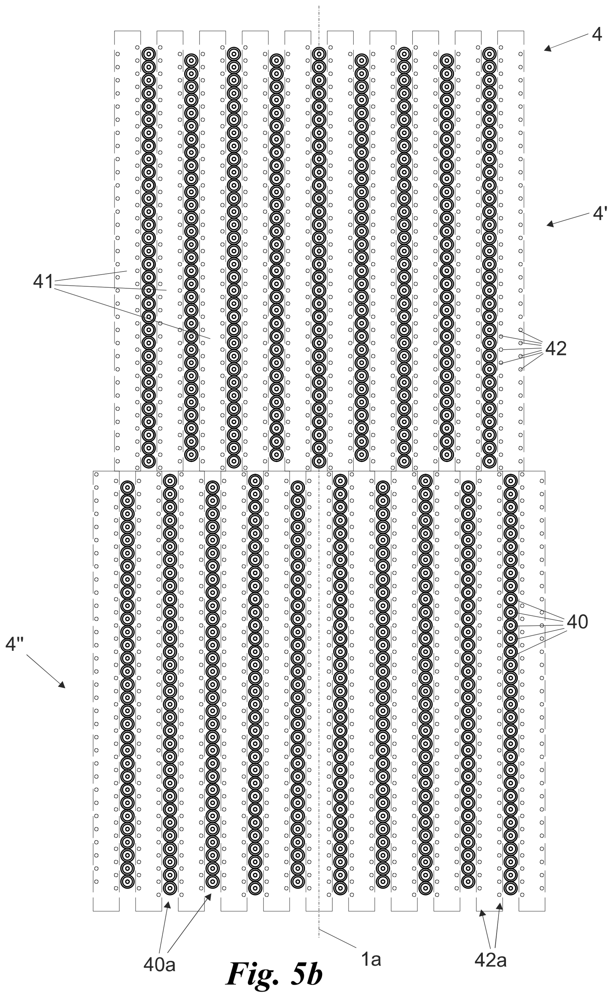

**Fig. 5b** illustrates an upper schematic view, i.e., from the point of view of the dispenser, of the first holes and slits and second holes, shown in hatches, of a spinneret of a plant to make nonwoven fabric according to the invention in which the slits are parallel to the main axis and staggered between a first portion and a second portion of the spinneret along the main axis;

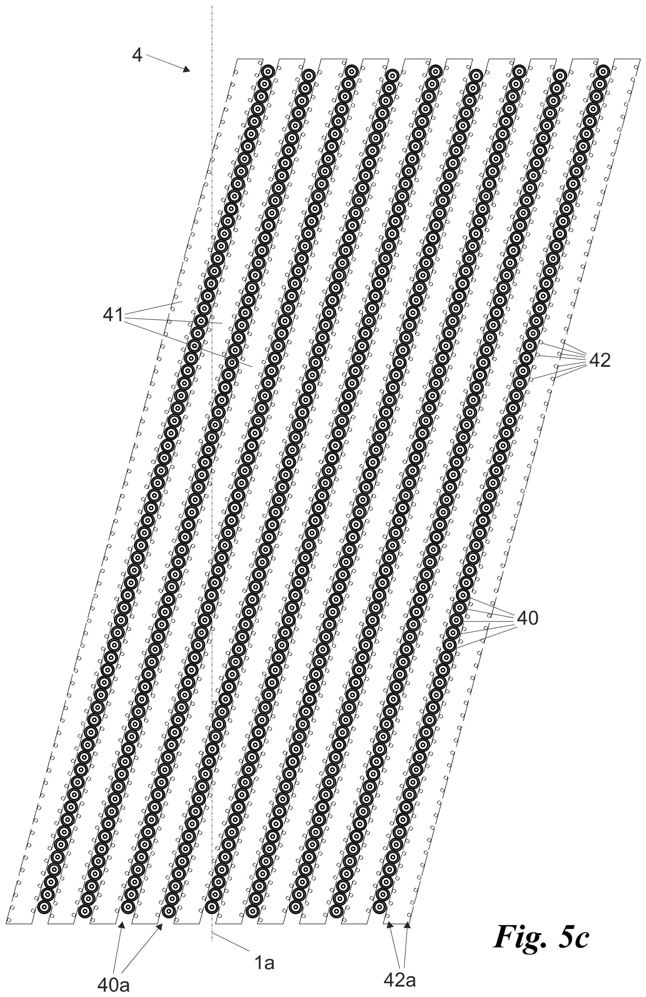

**Fig. 5c** illustrates an upper schematic view, i.e., from the point of view of the dispenser, of the first holes and slits and second holes, shown in hatches, of a spinneret of a plant to make nonwoven fabric according to the invention in which the slits are slightly inclined with respect to the main axis;

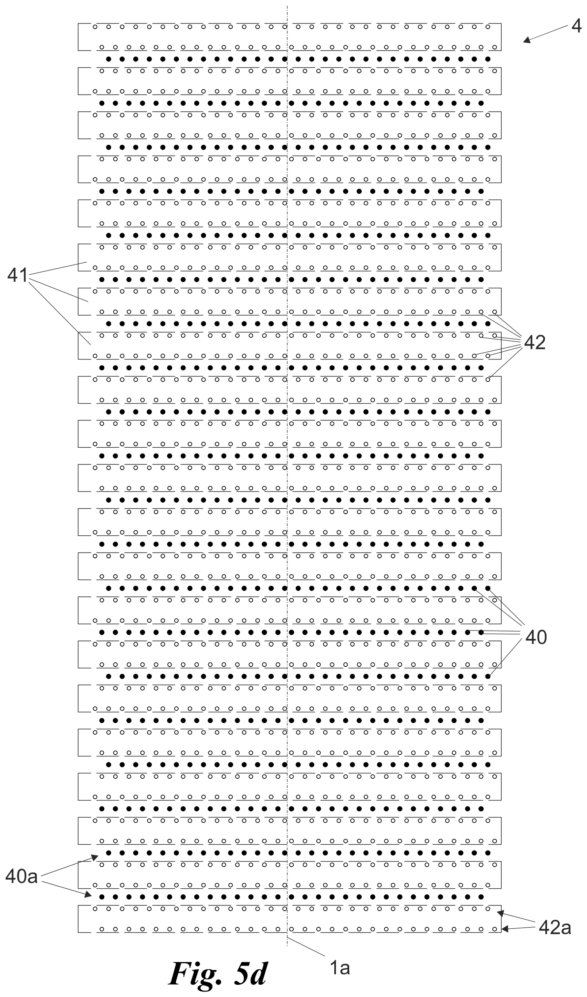

**Fig. 5d** illustrates a lower schematic view, i.e., from the point of view of the collecting surface, of the first and second holes and slits, shown in hatches, of a spinneret of a plant to make nonwoven fabric according to the invention in which the slits are perpendicular to the main axis;

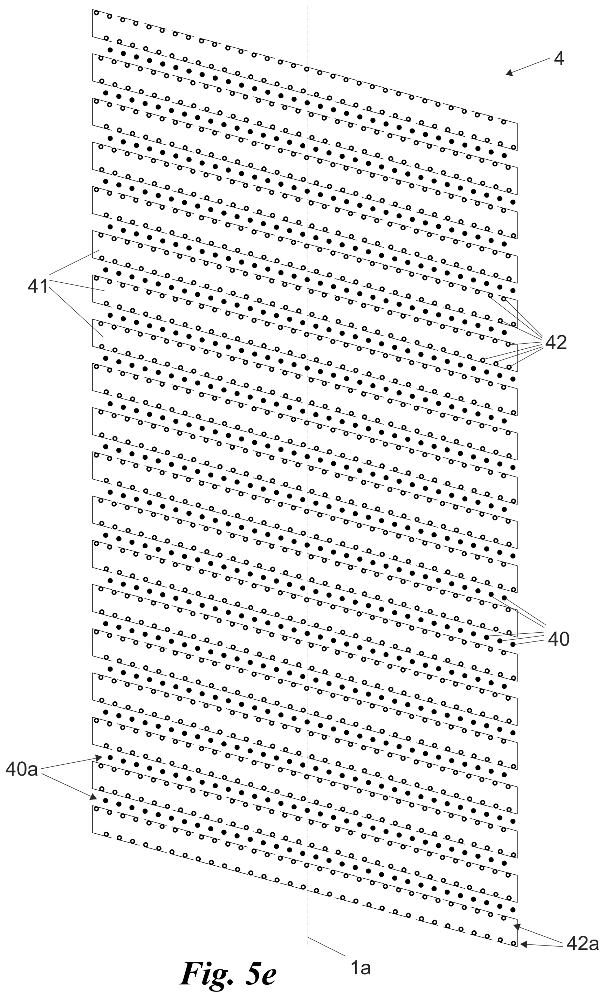

**Fig. 5e** illustrates a lower schematic view, i.e., from the point of view of the collecting surface, of the first and second holes and slits, shown in hatches, of a spinneret of a plant to make nonwoven fabric according to the invention in which the slits are strongly inclined with respect to the main axis;

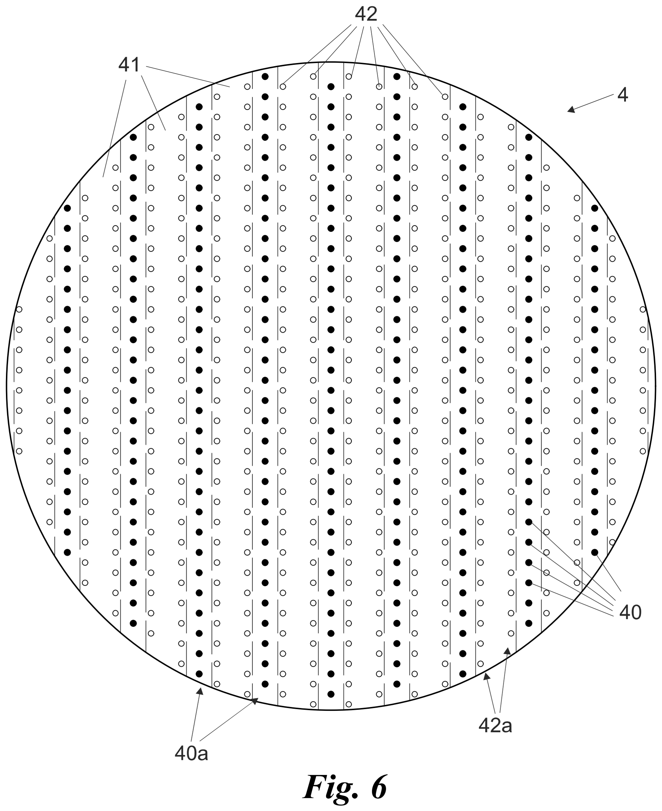

**Fig. 6** illustrates a lower schematic view, i.e., from the point of view of the collecting surface, of the first and second holes and slits, shown in hatches, of a circular spinneret of a plant to make nonwoven fabric according to the invention;

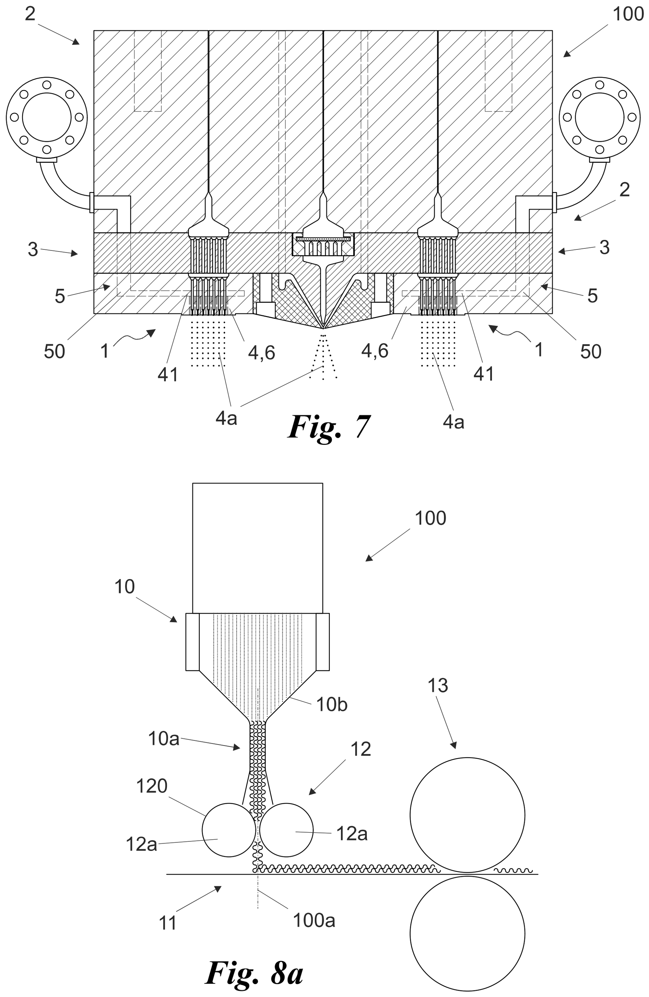

**Fig. 7** illustrates a cross-sectional view of an apparatus for making nonwoven fabric according to the invention in which a plant for making spunbond-type nonwoven fabric, a plant for making melt-blown nonwoven fabric, and a plant for making spunbond-type nonwoven fabric without the outer booth are sequentially present parallel to the main plane;
**Fig. 8a** shows a schematic view of an apparatus for making nonwoven fabric according to the invention in which the conveying station includes a pair of smooth rollers;

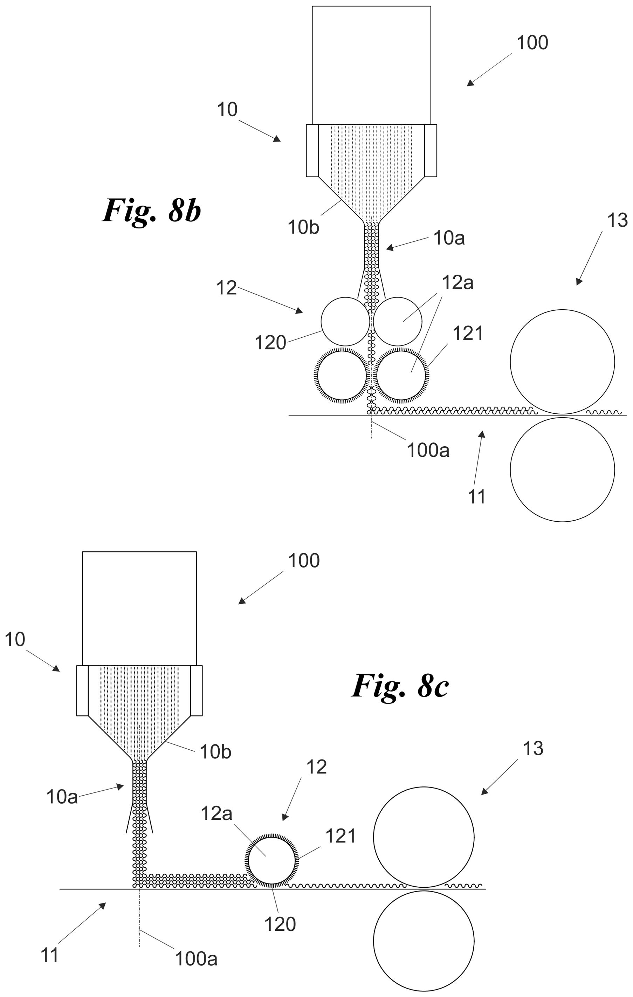

**Fig. 8b** shows a schematic view of an apparatus for making nonwoven fabric according to the invention in which the conveying station includes a first pair of smooth rollers and a second pair of rollers, downstream of the first pair, equipped with needles on the contact surface;
**Fig. 8c** shows a schematic view of an apparatus for making nonwoven fabric according to the invention in which the conveying station includes a pair of rollers equipped with needles on the contact surface placed close to the collection surface;

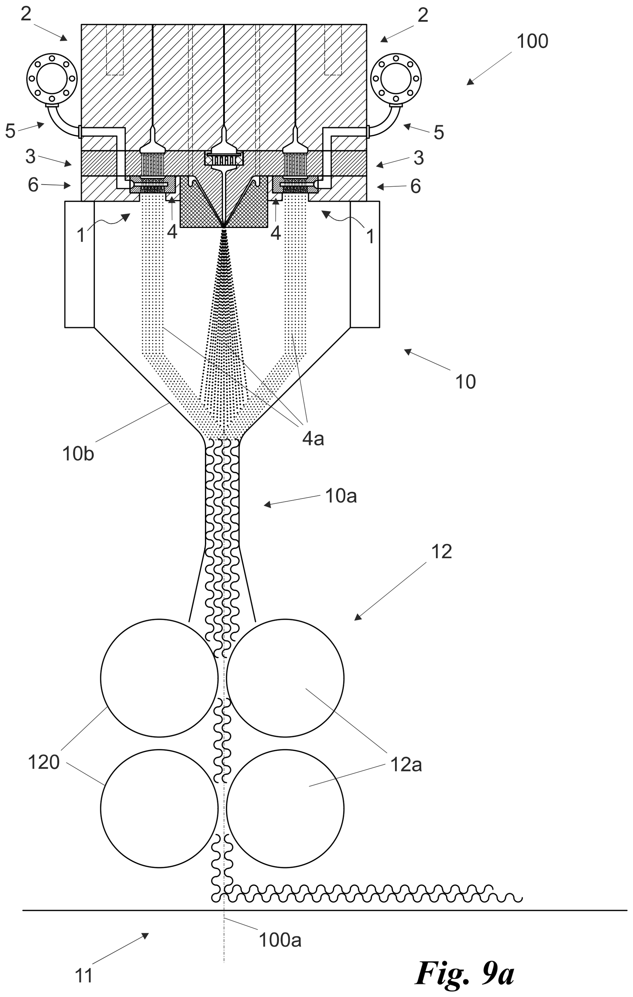

**Fig. 9a** illustrates a cross-sectional view of an apparatus for making nonwoven fabric according to the invention in which a plant for making spunbond-type nonwoven fabric, a plant for making melt-blown nonwoven fabric, and a plant for making spunbond-type nonwoven fabric are sequentially present in the booth parallel to the main plane and the conveying station includes two pairs of smooth rollers;

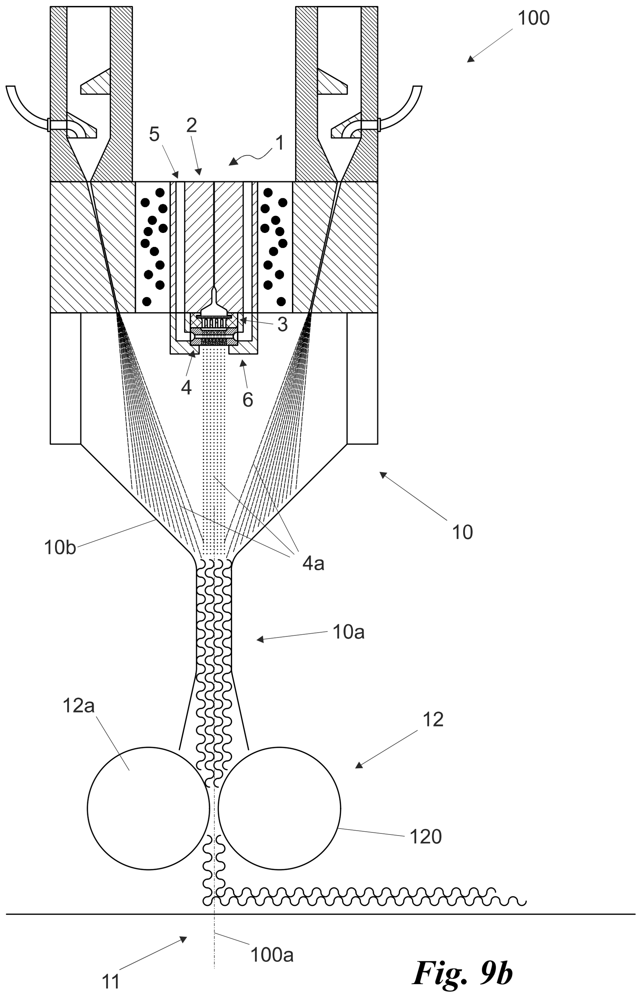

**Fig. 9b** shows a cross-sectional view of an apparatus for making nonwoven fabric according to the invention in which in the booth there are sequentially, parallel to the main plane, a plant for dispensing cellulose or viscose, a plant for making spunbond-type nonwoven fabric and a plant for dispensing cellulose or viscose, and the conveying station includes a pair of smooth rollers;

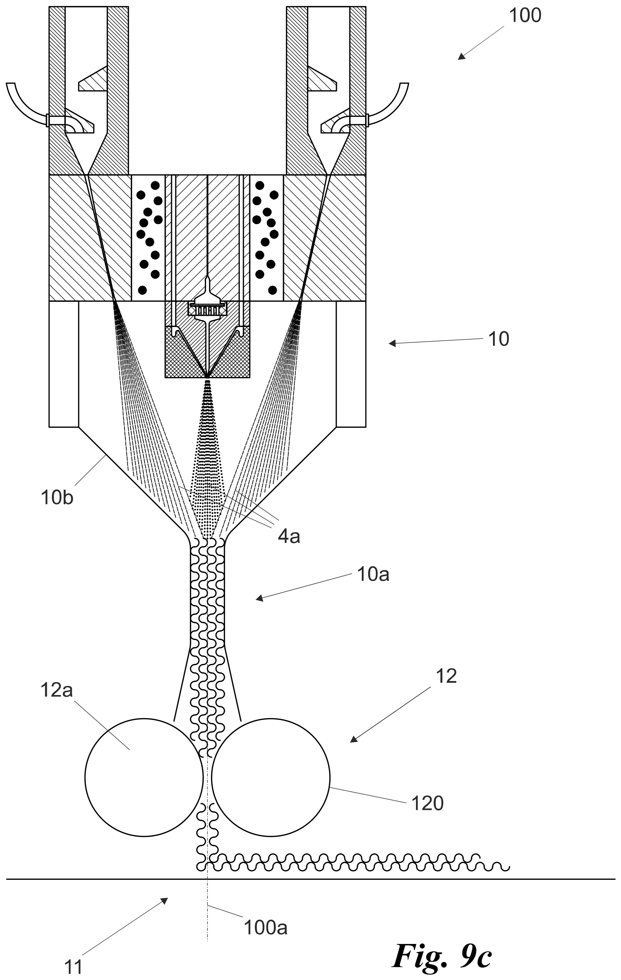

**Fig. 9c** shows a cross-sectional view of an apparatus for making nonwoven fabric according to the invention in which in the booth there are sequentially, parallel to the main plane, a plant for dispensing cellulose or viscose, a plant for making cusp melt-blown-type nonwoven fabric and a plant for dispensing cellulose or viscose, and the conveying station includes a pair of smooth rollers;

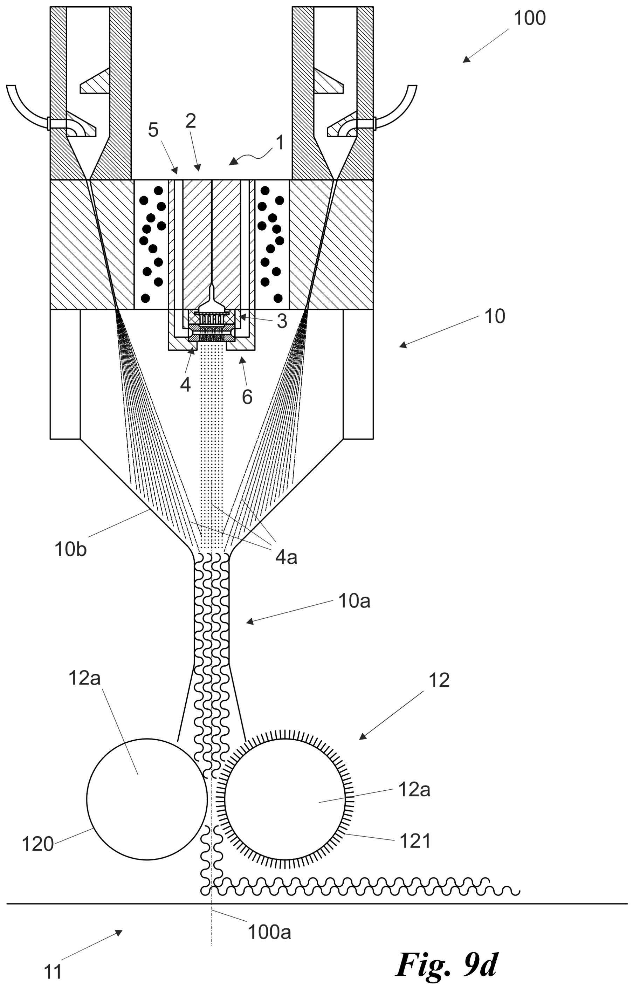

**Fig. 9d** shows a cross-sectional view of an apparatus for making nonwoven fabric according to the invention in which in the booth there are sequentially, parallel to the main plane, a plant for dispensing cellulose or viscose, a plant for making spunbond-type nonwoven fabric and a plant for dispensing cellulose or viscose, and the conveying station includes a pair of rollers of which one is smooth and one is equipped with needles;

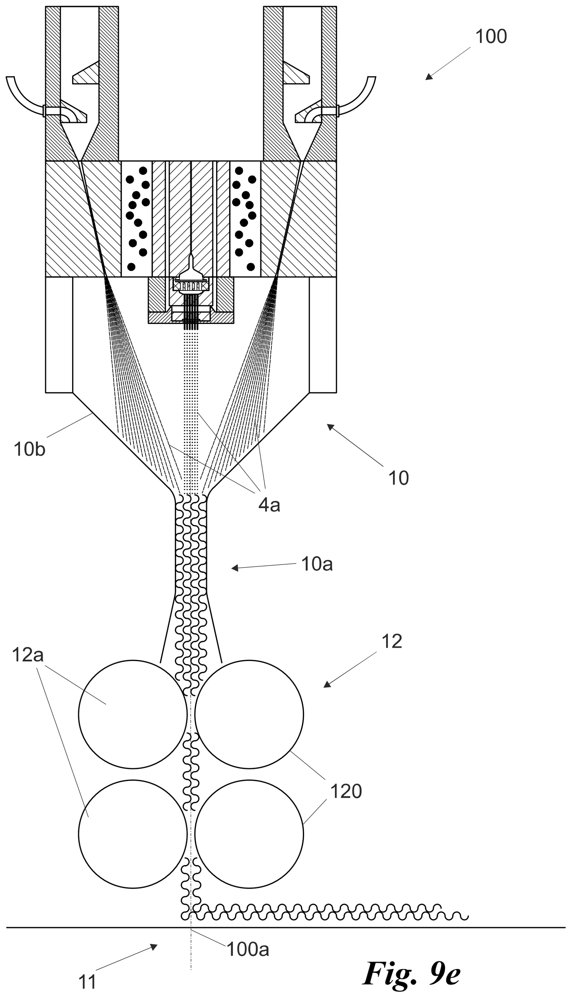

**Fig. 9e** shows a cross-sectional view of an apparatus for making nonwoven fabric according to the invention in which in the booth there are sequentially, parallel to the main plane, a plant for dispensing cellulose or viscose, a plant for making coaxial multi-row melt-blown-type nonwoven fabric and a plant for dispensing cellulose or viscose, and the conveying station includes two pairs of smooth rollers;

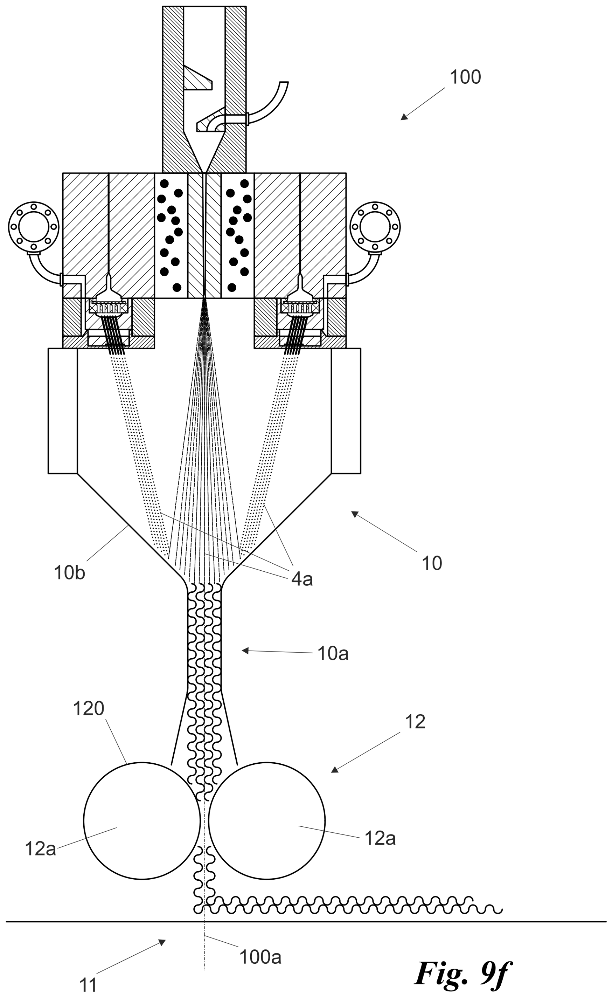

**Fig. 9f** illustrates a cross-sectional view of an apparatus for making nonwoven fabric according to the invention in which in the booth there are sequentially, parallel to the main plane, a plant for making multi-row coaxial melt-blown-type nonwoven fabric, a plant for dispensing cellulose or viscose, and a plant for making multi-row coaxial melt-blown melt-blown in which the melt-blown-type plants define dispensing directions converging with respect to the dispensing plane and the conveying station includes a pair of smooth rollers;

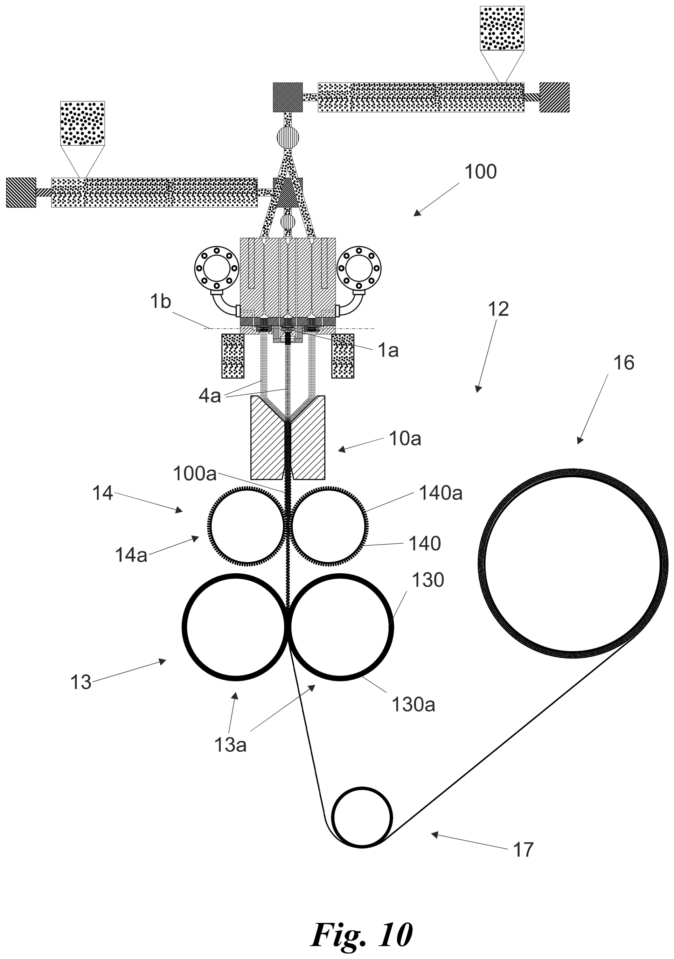

**Fig. 10** shows a schematic cross-sectional view of an apparatus for making nonwoven fabric according to the invention in which compacting and calendering means are present sequentially, parallel to the dispensing plane, in the absence of any belt or mat collection surface;

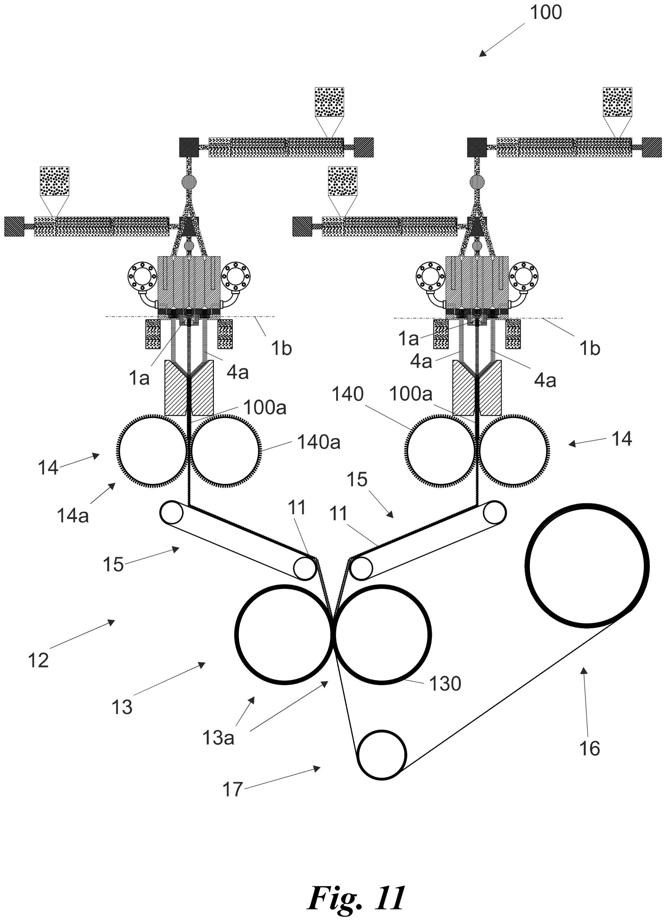

**Fig. 11** illustrates a schematic cross-sectional view of an apparatus for making nonwoven fabric according to the invention in which there are a plurality of distinct plants at the respective outlets of which are placed, in addition to the compacting means, respective conveyors for separate conveying of polymer filament layers to the calendering means;
**Fig. 12** illustrates a schematic cross-sectional view of an apparatus for making nonwoven fabric according to the invention in which there are a plurality of distinct plants at the respective outlets of which are placed, in addition to the compacting means, respective conveyors for separate conveying of polymer filament layers to the calendering means and in which at least one layer of filaments is conveyed by a plant to calendering means without the conveyor; and
**Fig. 13** depicts a schematic cross-sectional view of an apparatus for making nonwoven fabric of the prior art in which the presence of a conveyor belt between the calender outlet and rollers is evident.

In this document, when measurements, values, shapes, and geometric references (such as perpendicularity and parallelism) are associated with words like "approximately" or other similar terms, such as "almost" or "substantially", they are to be understood as excluding measurement errors or inaccuracies due to production and/or manufacturing errors and, above all, as having less than a slight deviation from the associated value, measurement, shape, or geometric reference. For example, if associated with a value, such terms preferably indicate a deviation by no more than 10% of the value itself.

Moreover, when used, terms such as "first", "second", "upper", "lower", "main" and "secondary" do not necessarily identify an order, a priority of relationship or a relative position, but can simply be used to clearly distinguish between their different components. Unless otherwise specified, as reflected in the following discussions, terms such as "processing", "computing", "determination", "computing", or the like are considered to refer to the action and/or processes of a computer or similar electronic computing device that manipulates and/or transforms data represented as physical, such as electronic quantities of records of a computer system and/or memories, in other data similarly represented as physical quantities within computer systems, records, or other information storage, transmission, or display devices.

Unless otherwise stated, the measurements and data reported in this text shall be considered as provided in International Standard Atmosphere ICAO (ISO 2533:1975).

With reference to the Figures, the plant to make nonwoven fabric according to the invention is globally referred to as number **1.**

Plant 1 to make nonwoven fabric according to the invention is preferably of the spunbond type.

Therefore, like any spunbond plant, plant 1 is at least capable of conveying polymer filaments onto a deposition surface to make nonwoven fabric.

It should be premised that, by nonwoven fabric, we mean any type of fabric where the filaments are deposited in bulk, that is, not sorted through a loom or other means by which they can be woven in weave, warp and weft, but are mixed into one or more layers of an item by deposition on a deposition surface, as further explained later. Therefore, nonwoven fabric can be made not only, as is conventionally done, from a polyester filament, but from any natural or synthetic material such as, for example, polypropylene, nylon, cellulose, viscose, and, of course, polyester itself.

Similarly, filament means any filiform element defining, in general, a chemical, acrylic and synthetic fiber.

It is also specified that plant 1 is capable of making nonwoven fabric, but it is also capable of making, more basically, only one or more filaments that can be used for nonwoven fabric formation or used in some other way, such as unreeled.

Preferably, plant 1 develops mainly along a main axis **1a.** The main axis 1a is a virtual axis, for example barycentric, along which the plant 1 extends.

In addition, plant 1 also extends along a main plane **1b.** The main plane 1b can be provided, for example, by an intermediate plane, preferably parallel to the surface on which the polymeric filaments that make up the nonwoven fabric are deposited.

The main axis 1a may be parallel and in some cases co-planar to the main plane 1b.

In addition, the plant 1 defines a vertical axis 1c.

The vertical axis 1c is preferably perpendicular to the main axis 1a. Hence, the vertical axis 1c is preferably also perpendicular to the main plane 1b. Therefore, the vertical axis 1c is preferably oriented perpendicular to the surface on which the polymeric filaments that make up the nonwoven fabric are deposited and runs along the plant 1 from upstream to downstream.

Thus, the vertical axis 1c is essentially an extrusion axis along which at least part of the polymeric fluid is conveyed to the dispenser within plant 1.

Plant 1 includes, like all spunbond-type plants, at least one extrusion head **2,** one dispenser **3** and one spinneret **4.**

Specifically, plant 1 includes extrusion head 2, dispenser 3 and spinneret 4, preferably in that order, along vertical axis 1c.

Then, the polymeric fluid is introduced into the extrusion head 2, then into the dispenser 3 and then distributed into the spinneret 4.

Preferably, the polymeric fluid is a choice of polypropylene, polyester, nylon, cellulose, polyester, and viscose.

Extrusion head 2 is preferably basically a common extrusion head, also known by the term *coat-hanger.*

Preferably, the extrusion head 2 is suitable for directing a polymeric fluid to the dispenser 3. Therefore, preferably, extrusion head 2 includes at least one main channel **20.**

Main channel 20 is an opening or conduit of any shape or size and can therefore be cylindrical or square compatible with the other elements to which main channel 20 interfaces.

Main channel 20 is preferably suitable for the passage of polymeric fluid through the extrusion head 2. Specifically, the main channel 20 is suitable for flowing the polymeric fluid to the dispenser 3. Of course, extrusion head 2 could also include a plurality of main channels 20.

Dispenser 3, as mentioned above, receives polymeric fluid from extrusion head 2, specifically from main channel(s) 20, to distribute it.

Therefore, dispenser 3 is preferably integral to extrusion head 2.

Dispenser 3 is also referred to by the term *breaker-plate.*

Therefore, it includes at least one dispensing duct **30.**

The dispensing duct 30 is in fluid passage connection with main channel 20 and is suitable for distributing polymeric fluid. In detail, dispenser 30 distributes the polymeric fluid to spinneret 4 to make the polymeric filaments that then go on to compose the nonwoven fabric.

Both the main channel 20 and the dispensing duct 30 can define complex shapes. In addition, they can split and provide sub-channels for distribution of the polymeric fluid. In other words, the dispenser 3 may also include a plurality of dispensing ducts 30 arranged downstream of the main channel 20 and designed, each, to distribute smaller amounts of polymeric fluid than the main channel 20. Preferably, the dispenser 3 includes a plurality of dispensing ducts 30 such that the polymeric fluid is distributed along the main plane 1b.

Dispenser 3 preferably includes at least one housing and filter means.

This housing is, preferably, adjacent to the extrusion head 2 and placed between the main channel 20 and the dispensing duct 30.

More specifically, the housing can be obtained on the dispenser 3 in the interface area with the extrusion head 2.

Thus, dispenser 3 may include a single housing or it may have a plurality of adjacent housings distributed along dispenser 3 transversely to vertical axis 1c.

Filter media are preferably housed in the housing.

The housing is, therefore, basically a tank inside which filter media can be placed. The latter are preferably arranged between the main channel 20 and the dispensingh ducts 30, i.e., preferably in the housing, in such a way as to filter the polymeric fluid.

Filter means, in particular, preferably include a porous element.

The porous element defines a plurality of non-rectilinear and irregularly sized passage channels.

It is basically structurally similar to a sponge. In this sense, it is not meant that the porous element is a soft element that can be easily deformed. On the contrary, the porous element is preferably defined in terms of stiffness and hardness by the materials and manufacturing processes that distinguish it.

However, the porous element also includes, like sponges, a plurality of pores that make the porous element itself substantially inhomogeneous and anisotropic.

In particular, preferably, the porous element is an element made by sintering technique. The latter, due to the manufacturing process, preferably includes a plurality of non-rectilinear passage channels of varying sizes.

The term sizes refers to all dimensions that contribute to the volumetric determination of the cavities or pores characterizing the porous element.

More in detail, preferably, filter means include a plurality of pores, or cavities, defining passageways when adjacent. In other words, the consequentiality of the pores realizes the passage channels for the polymeric fluid.

Also, preferably, each of the pores has a porosity greater than or equal to 18 µm.

As mentioned above, the porous element is preferably processed by sintering and, therefore, comprises a plurality of mutually sintered particles of the same material.

The particles can then be of any material, as long as it is resistant to the passage of the polymeric fluid. For example, the porous element includes metallic material.

In addition, the sintered particles can be of different natures.

For example, in a first embodiment, the particles can be components with a choice of spheres or fragments.

In a second embodiment, the porous element may include mutually intertwined and sintered filaments.

In a third embodiment, the porous element may include a first layer and a second layer. These layers are preferably mutually overlapping and include components and filaments, respectively, as previously described. In detail, preferably, the first layer and the second layer are adjacent to extrusion head 2 and dispenser 3, respectively, alternately they are adjacent to dispenser 3 and extrusion head 2, respectively.

In addition, single-layer sintering of both particles and filaments can be provided.

The porous element can then be made in one piece with dispenser 3 or, preferably, is removably available in dispenser 3, particularly in the housing.

Preferably, therefore, the porous element is countershaped apart from the housing and removably arranged within the housing.

Therefore, in cases where the housings are multiple, the dispenser 3 may include a plurality of porous elements that can be introduced as tablets within the appropriate housings.

Advantageously, each housing is adapted to accommodate the porous element in such a way that the latter is spaced from the dispensing duct 30. Preferably, especially when the dispenser 3 includes a plurality of dispensing ducts 30, the housing is configured to avoid adhesion between the filter means and the dispensing ducts 30.

Thus, in general, the housing is configured to distance the filter means and the dispensing duct 30 so that at least a separation space is realized.

The separation space preferably is a portion of the free space between the porous element and the dispensing duct 30 defining a thickness along the vertical axis 1c.

Preferably, the thickness of the separation space is greater than or equal to 500 µm. Even more preferably, the separation space is between 500 µm and 8 mm.

Preferably, but not necessarily, the separation space defines a thickness greater than or equal to the thickness of the porous element. The latter, in the preferred form of implementation, defines a thickness of at least 1.5 mm. Even more in detail, preferably, the porous element defines thicknesses between 1.5 mm and 8 mm.

To achieve separation between filter media and the inlet of dispensing duct 30, the housing may include support means.

The support means are preferably designed to allow the porous element to rest on them in such a way that the latter remains within the housing spaced from the dispensing duct(s) 30.

Therefore, the support means may include a support frame, or other spacer elements that can realize at least the separation space.

The spinneret 4 is a preferably separate element from dispenser 3, as in common spunbond plants. However, preferably, the spinneret 4 is at least integral to the dispenser 3. Preferably, the spinneret 4 comprises a plurality of first holes **40.** Each of the first holes 40 is preferably developed transversely to the main plane 1b.

In addition, each of the first holes 40 is preferably in fluid passage connection with the dispensing duct(s) 30 and suitable for extruding polymeric fluid to make a respective filament, preferably polymeric.

Appropriately, the first holes 40 may have diameters that vary dimensionally depending on the use for which they are intended.

In addition, the first holes 40 can be shaped circularly, or define other shapes transversely to their own development axis, that is, defined by cross sections of the first 40 holes of different shapes and sizes. For example, the first holes 40 can define a circular, quadrangular, triangular outline, with sharp or preferably beveled corners and also present concavity or convexity.

Advantageously, spinneret 4 of plant 1 according to the invention includes at least one slit **41.**

Slit 41 preferably runs parallel to the main plane 1b. Thus, slit 41 is suitable for allowing air to pass through spinneret 4.

Slit 41 can extend from side to side of spinneret 4. Or, slit 41 may also develop only partially within spinneret 4.

Thus, spinneret 4 also advantageously includes a group of second holes **42.**

The second holes 42 are in fluid passage connection with slit 41.

Then, the second holes 42 are developed parallel to the first holes 40 in such a way as to allow air to escape from slit 41.

Therefore, first and second holes 40, 42 develop along or parallel to one or more of the dispensig directions **4a** along which the polymer filament is delivered and the air that pushes the filaments outward from the plant 1.

The one or more dispensing directions 4a, therefore, correspond to the directions along which polymer filaments are dispensed from spinneret 4.

The second holes 42 are preferably distributed throughout spinneret 4 so that the air exiting spinneret 4, particularly from slit 41, can interact with the polymeric fluid exiting from all the first holes 40.

In addition, slit 41 can be unique and develop parallel to the main plane 1b such that it is in fluid passage connection with all the second holes 42.

Or, spinneret 4 may comprise a plurality of slits 41 in fluid passage connection, each, with respective groups of second holes 42.

In fact, preferably, the first holes 40 are organized into first rows **40a.**

The first rows 40a are basically mutually distinct, separate and parallel rows of first holes 40. Thus, the slits 41 are, in turn, preferably mutually distinct and separate. In addition, slits 41 run parallel to the first rows 40a and between the first rows 40a mutually adjacent to each other.

In even more detail, the slits 41 are developed in such a way that they do not interfere with the first holes 40. Furthermore, advantageously, spinneret 4 includes pluralities of groups of second holes 42 each in fluid passage connection with a respective slit 41.

In addition, spinneret 4 can define specific shapes and sizes consistent with the structure of plant 1 and, for example, the dispenser.

So, for example, spinneret 4 can define a rectangular shape, or more generally a quadrangular shape, or even a rounded shape, such as circular.

Thus, spinneret 4 can determine specific orientations of the first rows 40a, and thus also of the slits 41, with respect to the main axis 1a.

For example, in a first embodiment shown in Figs. 5a-5b and 6, the first rows 40a can develop parallel to the main axis 1a.

Or, as shown in Figs. 5c-5e, the first rows 40a can develop transversely to the main axis 1a such that they are perpendicular (Fig. 5d) to the main axis 1a or oblique (Figs. 5c and 5e) with respect to the main axis 1a.

In any form of implementation, spinneret 4 can be divided into portions or regions. For example, spinneret 4 may include a first portion **4'** and a second portion **4".**

If present, first and second portions 4', 4" include respective first rows 40a mutually staggered such that each first row 40a of the first portion 4' is aligned with a respective slit 41 of the second portion 4" and that, conversely, each first row 40a of the second portion 4" is aligned with a respective slit 41 of the first portion 4', as for example shown in Fig. 5b. Second holes 42 can also be arranged in specific ways.

For example, advantageously, the second holes 42 of one or more among the groups of second holes 42, preferably of each of the groups, are arranged in at least one pair of second rows **42a.**

The second rows 42a are mutually distinct, separate and parallel. Thus, the second rows 42a are preferably each placed near a respective first row 40a of two adjacent first rows 40a.

Also, advantageously, the second holes 42 of each second row 42a are preferably arranged offset from the first holes 40 of an adjacent first row 40a so that they are positioned, transversely to the first row 40a, between two adjacent first holes 40 of the first row 40a.

In this way, the second holes 42 allow air to be delivered, coming out of spinneret 4, surrounding all the filaments coming out of the first holes 40.

Since slit 41 is suitable for allowing the passage of air, preferably plant 1 also includes conveying means **5.**

Conveying means 5 are in fluid passage connection with slit 41. Thus, conveying means 5 are configured to convey air into slit 41.

The air conveyed is, therefore, replacement air that therefore allows the first holes 40, and consequently the polymeric fluid, to be cooled, at least externally, before the polymeric fluid escapes from spinneret 4.

In addition, since air also escapes through the second holes 42 adjacent to the first holes 40, it also directly cools the polymeric fluid immediately at the exit of spinneret 4 and also between the first holes 40, as explained above.

This technical aspect is very advantageous as later better explained.

In addition to what is described, plant 1 may include additional details.

As mentioned above, spinneret 4 is preferably separated from dispenser 3 and can define, for example, quadrangular (Fig. 3a) or circular shape (Fig. 3b)

Thus, plant 1 may further include a support **6.**

Support 6, shown for example in Figs. 1-2 and 4, if any is integral to dispenser 3. Thus, support 6 is suitable for supporting the spinneret 4 in such a way as to keep it integral to the dispenser 3.

In this regard, preferably support 6 includes a housing **60.**

Housing 60 is perforated and accommodates spinneret 4 without obstructing holes 40, 42. Therefore, in turn, preferably conveying means 5 include one or more conveying ducts **50.** The one or more conveying ducts 50 are preferably developed between extrusion head 2, dispenser 3 and support 6. In addition, conveying ducts 50 are in fluid passage connection with one or more slits 41, preferably either one with all slits 41, or each with the same slit 41 or a reciprocal slit 41.

Plant 1, in conclusion, preferably includes extrusion head 2, dispenser 3 and support 6 mutually distinct, separate and, in that order, mutually constrained. Thus, spinneret 4 is removably constrained to support 6 trapped in housing 60 between dispenser 3 and support 6.

Plant 1, therefore, is capable of delivering filaments, preferably polymeric, along and parallel to the dispensing direction 4a, which can be parallel to the vertical axis 1c or even inclined with respect to it.

Preferably, plant 1 is used to make nonwoven fabric. Then, preferably, the filaments are deposited on a deposition surface, which can be movable, so that at least one layer of interlacing filaments is formed during deposition.

However, plant 1 could be equivalently employed to make a filament(s) reel. In this regard, in fact, it could be planned to place a reel tangent to one or more of the dispensing directions 4a, for example, developing parallel to the main axis 1a and rotating around an axis parallel to it, so that filament is collected on the rotating reel.

In this way, the filament could also be used for true weaving, employing weaving looms that can unwind the filament and weave it into warp and/or weft.

Similarly, the invention does not only include a new plant 1.

In fact, the invention also makes it possible to make new equipment.

Specifically, the invention enables at least one new apparatus for making nonwoven fabric, which, with reference to the Figures, is globally referred to as **100.**

It is important from the outset to explain that apparatus 100 may include a plant 1, or it may not.

In addition, the apparatus 100 can be configured according to different modes and, more specifically, according to a plurality of different possible advantageous configurations.

For example, among the various embodiments, apparatus 100 may include, like most apparatus involving spunbond or melt-blown plants, a booth **10.**

If present, booth 10 is basically a containing enclosure within which, normally, a plant capable of dispensing filaments is housed, such as an plant 1. In the example where plant 1 is in booth 10, the latter includes all the components of plant 1, such as extrusion head 2, dispenser 3, spinneret 4, possibly also support 6, and conveying means 5. Or, booth 10 can also include, in this example, only spinneret 4, possibly support 6, in order to accommodate filament in an enclosed environment.

Booth 10 defines, therefore, a closed containment volume, i.e., inaccessible from the outside.

In addition, plant 1 includes an outlet **10a.**

In general, plant 1 is in fact configured to deliver via outlet 10a a plurality of filaments of the type chosen from polymeric or cellulose or viscose.

Preferably, delivery is accomplished by venturi effect.

Thus, dispensing preferably occurs along and parallel to the one or more dispensing directions 4a transverse to the main plane 1b.

If booth 10 is present, the latter may include outlet 10a.

Outlet 10a can then correspond to the portion of booth 10 from which the filament(s) dispensed by the plant present in booth 10 can escape.

Since normally spunbond or melt-blown plants, as well as other plants suitable for dispensing filaments, are three-dimensional objects that are developed along a plane transverse to the dispensing direction 4a, analogous to the main plane 1b of plant 1, outlet 10a is, therefore, essentially developed along a dispensing plane **100a.** The dispensing plane 100a is a virtual plane, preferably transverse to main axis 1a and main plane 1a, along which, or parallel to which, filaments are dispensed.

Thus, the filaments exiting the outlet 10a preferably form a filament layer along or parallel to the dispensing plane 100a.

Thus, outlet 10a can structurally define a dispensing slit whose area extends transversely to the dispensing plane 100a.

In fact, outlet 10a preferentially defines a volume narrowing configured to allow a plurality of filaments to be dispensed from plant 1, e.g., from booth 10. Shrinkage, as such, allows filaments in particular to be dispensed by venturi effect by forming, at the outlet, a set of filaments that results, as mentioned above, in the formation of the filament layer along or parallel to the dispensing plane 100a.

Regardless of the presence, or absence, of booth 10, plant 1 preferably also includes a funnel portion **10b.**

Funnel portion 10b is preferably placed upstream of outlet 10a. Then, the funnel portion 10b converges to direct filaments to outlet 10a.

This means that, for example, when plant 1 includes booth 10, filaments are deposited on the walls of booth 10 at the funnel portion 10b, sliding independently to outlet 10a.

In an embodiment, apparatus 100 may additionally include one or more collection surfaces **11.**

The collection surface 11 may substantially correspond to the surface previously referred to as the deposition or deposition surface. Thus, the collection surface 11 runs transversely to the dispensing plane 100a downstream of the outlet 10a. Collection surface 11 is configured, therefore, to collect the output layer from outlet 10a so as to make a nonwoven fabric article.

As mentioned above, apparatus 100 may include a plant 1 which, as described, is of the spunbond type.

More generally, apparatus 100 can include any plant to make nonwoven fabric. The latter is, in apparatus 100, possibly but not necessarily housed in booth 10.

Thus, the plant is developed along a main axis 1a and a main plane 1b that are transverse to the dispensing plane 100a. In addition, the plant is configured to dispense toward outlet 10a at least one filament of the type of your choice of polymeric or cellulose or viscose along one or more dispensing directions 4a transverse to the main plane 1b.

In an first embodiment of apparatus 100, the latter may include, or be lacking, booth 10.

In fact, preferably apparatus 100 includes at least one plant as previously generically described and one conveying station **12.**

Thus, to summarize, the plant is along a main axis 1a and a main plane 1b and is configured to dispense, preferably but not necessarily by venturi effect especially if booth 10 is also present, via an outlet 10a a plurality of any type of polymeric or cellulose or viscose filaments along and parallel to one or more dispensing directions 4a transverse to main plane 1b forming, at the outlet from outlet 10a, a layer of filaments along or parallel to dispensing plane 100a.

Conveying station 12, on the other hand, is preferably located downstream of outlet 10a. Conveying station 12 is basically suitable for conveying the filament layer outside the plant. So, for example, apparatus 100 might include a winder **16.**

If present, winder 16 could be configured to allow the layer to be wound onto itself to form a reel. Therefore, conveying station 12 could be configured to convey the output layer from outlet 10a to winder 16.

Of course, the path between conveying station 12 and winder 16 could be facilitated by the presence of additional elements. In fact, apparatus 100 could also include transfer devices **17.**

If present, transfer devices 17 are configured to route the layer from conveying station 12 to winder 16. They may, for example, include one or more secondary rolling elements, for example, suitable for holding the sliding belt by means of vacuum systems.

Advantageously, in general, the conveying station 12 includes at least calendering means **13.**

Calendering means 13 are means to enable the layer to be calendered, which, as is well known, is an industrial process by which the layer is slid within a gap, such as between two rolling wheels, to compress and stretch the layer by reducing its local thickness.

The calendering means 13 are, therefore, advantageously spaced from the outlet 10a by a collection distance of between 0 m and 5 m. The collection distance could also be less, for example between 0.1 m and 3 m, even more in detail preferably 0.5 m and 1.5 m, more appropriately 1 m.

The conveying station 12 in this form of implementation advantageously lacks a collection surface, such as the belt or mat collection surface 11 placed upstream of the calendering means 13. This means that, as clearly shown in Fig. 10, apparatus 100 allows the layer to be conveyed out of the plant in such a way that the layer is directly calendered into the calendering means 13 without being deposited on the belt or mat collection surface beforehand.

Avoiding the deposition of filaments allows the strength of the nonwoven layer to be increased and, in addition, it also allows nonwoven articles to be made without layering the various uncompressed layers on a deposition surface, but appropriately possibly combining them only after calendering.

Of course, to further improve the plant, it can also include additional components.

For example, conveying station 12 may also include compacting means **14.**

If present, compacting means 14 are placed between outlet 10a and calendering means 13. Then, the compacting means 14 are configured to compress the layer in order to interlace and weld the layer strands together before the layer is conveyed to the calendering means 13.

Thus, preferably, compacting means 14 exert more pressure, compared to calendering means 13, on the filament layer.

The calendering means 13 and/or compacting means 14 could, therefore, include additional details.

For example, calendering means 13 may include a pair of first rollers **13a.**

If present, the first rollers 13a are preferably counter-rotating. In addition, the first rollers 13a run parallel to the main plane 1b. Thus, they are rotating around respective rotation axes oriented with respect to the main axis 1a and configured to convey the layer away from the outlet 10a under pressure.

Of course, the first roller 13a can be fixed or selectively pivoted relative to the main axis 1a. The first roller 13a even more in detail includes a first contact surface **130.**

The first contact surface 130 is the portion of the first roller 13a suitable for contacting the filament(s) exiting outlet 10a.

The first contact surface 130 can, therefore, be smooth.

Or, the first contact surface 130 may include one or more knurls. If present, the knurls are configured to pick up filaments from outlet 10a along the dispensing plane 100a when the filaments contact the first contact surface 130. In addition, if present, the knurls are also configured to release the filaments in an orderly manner when the layer releases from the first contact surface 130.

In a particularly advantageous embodiment of the apparatus 100, the first contact surface 130 preferably includes a plurality of first needles **131.**

The first needles 131 are, if any, distributed radially along and around the axis of rotation. In addition, the first needles 131 protrude from the first contact surface 130.

Therefore, given the conformation, the first needles 131 are configured to punch the nonwoven layer delivered from outlet 10a so as to form distributed punches on the layer. Advantageously, at the punches, the filaments are then interlaced and welded to each other. Similarly, compacting means may include a pair of second rollers **14a.**

If present, the second rollers 14a are preferably counter-rotating. In addition, the second rollers 14a run parallel to the main plane 1b. Thus, they are rotating around respective rotation axes oriented with respect to the main axis 1a and configured to convey the layer away from the outlet 10a under pressure.

Of course, the second roller 14a can be fixed or selectively pivoted relative to the main axis 1a.

The second roller 14a even more in detail includes a second contact surface **140.**

The second contact surface 140 is the portion of the second roller 14a suitable for contacting the filament(s) exiting outlet 10a.

The second contact surface 140 can, therefore, be smooth.

Or, the second contact surface 140 may include one or more knurls. If present, the knurls are configured to pick up filaments from outlet 10a along the dispensing plane 100a when the filaments contact the second contact surface 140. In addition, if present, the knurls are also configured to release the filaments in an orderly manner when the layer releases from the second contact surface 140.

In a particularly advantageous embodiment of the apparatus 100, the second contact surface 140 preferably includes a plurality of second needles **141.**

The second needles 141 are, if any, distributed radially along and around the axis of rotation. In addition, the second needles 141 protrude from the second contact surface 140. Therefore, given the conformation, the second needles 141 are configured to punch the nonwoven layer delivered from outlet 10a so as to form distributed punches on the layer. Advantageously, at the punches, the filaments are then interlaced and welded to each other. Calendering means 13 and compacting means 14 can also be arranged according to a particular conformation; in fact, preferably, calendering means 13 and compacting means 14 are placed sequentially along the dispensing plane 100a in such a way as to convey the layer along the dispensing plane 100a.

The configuration of apparatus 100 according to the invention just described enables a new process of making a nonwoven article.

The process is, of course, implemented by apparatus 100.

Thus, the process advantageously includes at least one conveying stage.

In the conveying stage, preferably, the layer is conveyed to the calendering means 13, from outlet 10a, without depositing the layer beforehand on a belt or mat collection surface. Even more in detail, advantageously, in the conveying stage, the layer could likewise be compacted, possibly punched so as to form punctures distributed over the layer at which the filaments are interlaced and sealed to each other.

Thus, the process also advantageously includes a calendering stage.

In the calendering stage, the layer is calendered with calendering means 13.

Of course, the invention also includes a nonwoven article made by a process as just described.

The embodiment of apparatus 100 just described can also be modified.

In fact, in a second embodiment, apparatus 100 preferably includes a plurality of distinct plants and conveying station 12.

As before, in summary, each plant is developed along a main axis 1a and a main plane 1b that are transverse to the dispensing plane 100a. In addition, the plant is configured to dispense toward outlet 10a at least one filament of the type of your choice of polymeric or cellulose or viscose along one or more dispensing directions 4a transverse to the main plane 1b. In addition, the plant may or may not be equipped with booth 10. Since the plants are preferably distinct, they are preferably not arranged within the same booth 10.

Thus, the conveying station 12 preferably includes the calendering means 13. The latter may or may not include the detail components described above, namely first rollers 13a, first contact surface 130 and first needles 131.

Advantageously, in this second form of implementation, the conveying station 12 also includes one or more conveyors **15.**

The one or more conveyors 15 are each placed between a respective outlet 10a and the calendering means 13. Thus, for example, apparatus 100 may include a conveyor 15 placed between one plant's outlet 10a and calendering means 13 and no conveyor 15 between outlet 10a of the other plant and calendering means 13, as for example shown in Fig. 12. Or, as shown in Fig. 11, apparatus 100 may include a conveyor 15 for each outlet 10a of the respective plant upstream of the calendering means 13.

Advantageously, the one or more conveyors 15 are each configured to convey a respective layer, separately from other layers, to the calendering means 13. In particular, they are configured in such a way that the layers are layered together exclusively at the calendering means 13.

Thus, in this second embodiment, it is designed to employ the same calendering means 13 to calendering a plurality of different layers of filaments dispensed from different plants, starting from the respective outlets 10a, avoiding that there are stratifications of the different layers on the same surface before calendering 13.

To improve, again, the performance of the article exiting the calendering means 13, possibly conveyed to the winder 16 via the transfer devices 17, the apparatus 100 can also include compacting means 14.

If present, compacting means 14 are placed downstream of one or more outlets 10a. Thus, if present, compacting means 14 are each placed between outlet 10a and calendering means 13. Then, they are configured to compress the single layer to interlace and seal the filaments to each other before the layer is conveyed to the calendering means 13.

Even more specifically, advantageously, the one or more conveyors 15 are placed between compacting means 14 and calendering means 13 so that already compacted layers can be conveyed.

Compacting means 14 can also include the details previously described, namely second rollers 14a, second contact surface 140 and second needles 141.

From a structural point of view, on the other hand, the one or more conveyors 15 may each comprise a collection surface 11. Collection surface 11, as mentioned above, runs transversely to the dispensing plane 100a downstream of outlet 10a to collect the layer exiting outlet 10a. Then, the collection surface 11 is configured to convey the layer to the calendering means 13. For example, the collection surface 11 can be made by a moving belt or mat. Or, the one or more conveyors 15 may be made by chutes or rollers or other elements that allow a path to be determined by which the layer can reach the calendering means 13 separately from the other layers.

In addition, again, the one or more conveyors 15 can be uniquely positioned with respect to the calendering means 13.

In fact, preferably, the calendering means 13 are spaced with respect to at least one of the one or more conveyors 15. In addition, the at least one of the one or more conveyors 15 is placed overhanging the calendering means 13.

Also the configuration of apparatus 100 according to the invention just described enables a new process of making a nonwoven article.

The process is, of course, implemented by apparatus 100.

Thus, the process advantageously includes at least one conveying stage.

In the conveying stage, preferably, each of the layers is conveyed separately from other layers from outlet 10a to the calendering means 13. Therefore, due to the different paths preferentially defined by the conveyors 15, the different layers are layered together exclusively at the calendering means 13.

Even more in detail, advantageously, in the conveying stage, the layer could likewise be compacted by compacting means 14, possibly punched so as to form punctures distributed over the layer at which the filaments are interlaced and sealed to each other.

Thus, the process also advantageously includes a calendering stage.

In the calendering stage, the layers are calendered together with calendering means 13. Of course, the invention also includes a nonwoven article made by a process as just described.

In a further of inventive embodiment of apparatus 100, the latter preferably includes booth 10 and additional plants in booth 10.

In fact, preferably, booth 10 includes at least two plants distributed parallel to the main plane 1b side by side. Of the two plants, also, preferably only one is of the spunbond or melt-blown type.

Therefore, given the configuration, at least two distinct types of filaments delivered by the plants are mixed simultaneously in booth 10 at the outlet 10a.

In this way, filaments of different types make one and the same layer at the output of booth 10.

This is very important because, normally, in common apparatuses the different filaments are not mixed before leaving the booth 10, but are instead layered on a collection surface 11 by means of several booths equipped with single arrangement equipment in succession. In a particularly advantageous form of implementation, preferably, booth 10 includes at least three plants distributed parallel to the main plane 1b such that at least three filaments, at least two of which are of different types, are mixed simultaneously in booth 10.

Booth 10 plants can, therefore, be configured in different ways.

First, the one or more dispensing directions 4a of each plant are either parallel to the dispensing plane 100a or may be converging to the dispensing plane 100a. This means that, for example, in an apparatus 100 wherein there are three plants, they may include a central plant with dispensing direction 4a parallel to or aligned with the dispensing plane 100a and side plants whose dispensing directions 4a converge toward the dispensing plane 100a and are therefore incident with and inclined with respect to the dispensing direction 4a of the central plant.

Of course, since plants can deliver a plurality of filaments, and the delivery can be linear or conical, each plant can define, as for example shown in Figs. 9a-9f of the dispensing cones within which the dispensing directions 4a are confined.

In addition, plants can define specific type configurations.

For example, in a embodiment shown in Figs. 7 and 9a, booth 10 may include, in that order, a spunbond type plant, a melt-blown type plant, and a spunbond type plant. This configuration, allows SMS-type layers to be obtained by simultaneous blending of filaments particularly advantageous for making diapers.

Or, in a embodiment shown in Figs. 9b-9e, booth 10 may include, in that order, a cellulose or viscose dispensing plant, a spunbond or melt-blown type plant, and a cellulose or viscose dispensing plant.

Cellulose or viscose dispensing plants are, similarly to the common melt-blown and spunbond plants, of themselves already known and are therefore not described in detail here.

This configuration allows absorbent layers to be obtained by simultaneous blending of filaments particularly advantageous for making wet wipes or similar.

Or again, in an embodiment shown in Fig. 9f, booth 10 may include, in that order, a melt-blown or spunbond type plant, a cellulose or viscose dispensing plant, and a melt-blown or spunbond type plant.

In case there are one or more melt-blown plants in booth 10, one of them can be of the cusp type (Fig. 9a) or multi-row coaxial type (Figs. 9e-9f).

In addition, in cases where there are one or more spunbond type plants in booth 10, they are preferably, but not necessarily, configured as plant 1 according to the invention.

The configurations of apparatus 100 according to the invention just described enable a new process of making a nonwoven article.

The process is, of course, implemented by apparatus 100.

Thus, the process advantageously includes at least one mixing stage.

At the mixing stage, preferably, at least two separate types of filaments dispensed from the plants at the outlet 10a in booth 10 are mixed simultaneously.

As mentioned above, filaments can also be mixed simultaneously in numbers of three.

The process, then includes a dispensing phase. In the dispensing stage, a single layer made by the mixed filaments of different types is dispensed from booth 10 through outlet 10a.

Thus, the process includes a deposition phase. In the deposition stage, the layer is collected on a collection surface 11 or on a reel. Thus, the layer deposited in this way enables the creation of a nonwoven article with greatly improved mechanical properties.

Of course, the invention also includes the article made by a process as just described.

Apparatus 100 according to the invention can also be configured differently.

For example, apparatus 100 may include booth 10 as described, collection surface 11, and also only one plant configured to dispense toward outlet 10a at least one filament of the type of your choice of polymeric or cellulose or viscose along one or more dispensing directions 4a transverse to the main plane 1b.

All these features have already been described, in detail, above.

However, in a further inventive form, apparatus 100 can also include a conveying station 12.

Conveying station 12 is placed, in this case, preferably between booth 10 and collection surface 11. In particular, in a preferred embodiment, conveying station 12 is also placed next to outlet 10a so as to, advantageously, close outlet 10a.

Or, in an alternative embodiment, or even in combination with the previous embodiment, the conveying station 12 is placed next to the collection surface 11.

Thus, conveying station 12 includes at least one rolling element **12a.**

The rolling element 12 preferably runs parallel to the main plane 1b. Thus, the rolling element 12 is preferably rotating about an oriented axis of rotation, e.g., parallel or perpendicular or oblique to the main axis 1a. Therefore, advantageously, the rolling element 12a is configured to convey the filaments of the filament layer in an orderly manner onto the collecting surface 11, particularly by orienting the filaments perpendicularly or parallel to the collecting surface 11.

Of course, the rollling element 12a can be fixed or selectively pivoted relative to the main axis 1a.

In addition, rolling element 12a may include, or consist of, the first roller 13a or the second roller 14a.

The rolling element 12a, even more in detail, includes an additional contact surface **120.** The latter may also include, or consist of, first contact surface 130 and second contact surface 140.

The further contact surface 120 is the portion of the second rolling element 12a suitable for contacting the filament(s) exiting outlet from booth 10

The further contact surface 120 can, therefore, be smooth.

Or, the further contact surface 120 may include one or more knurls. If present, the knurls are configured to pick up filaments from outlet 10a along the dispensing plane 100a when the filaments contact the further contact surface 120. In addition, if present, the knurls are also configured to release the filaments in an orderly manner when the layer releases from the further contact surface 120.

In a particularly advantageous embodiment of the apparatus 100, the further contact surface 120 preferably includes a plurality of additional needles **121.** Additional needles 121 may also correspond to first needles 131 or second needles 141.

The additional needles 121 are, if present, distributed radially along and around the axis of rotation. In addition, the additional needles 121 protrude from the additional contact surface 120.

Therefore, given the conformation, the additional needles 121 are configured to punch the nonwoven layer delivered from outlet 10a so as to form distributed punches on the layer. Advantageously, at the punches, the filaments are then interlaced and sealed to each other. Conveying station 12 of apparatus 100 can also be configured in different ways.

For example, in a first embodiment shown in Figs. 8a-8b and 9a-9f, the conveyor station 12 preferably includes at least one pair of mutually counter-rotating rolling elements 12a. In addition, the rolling elements 12a are configured so that the layer transits between the rolling elements 12a before being deposited on the collection surface 11.

In a further embodiment, as shown in Figs. 8b, 9a and 9e, conveying station 12 can include two pairs of rolling elements 12a.

In addition, the rolling elements 12a of a pair may include an identical additional contact surface 120. Or, as in the embodiment of Fig. 9d, the rolling elements 12a of a pair may include a different additional contact surface 120, particularly for example, one smooth and one including needles, or even one smooth and one knurled or one knurled and one including needles.

In the embodiment with single rolling element 12a, especially if the latter is as shown in Fig. 8c placed close to the collecting surface 11, the rolling element 12a preferably includes an additional contact surface 120 equipped with additional needles 121.

Apparatus 100 as just described, in conclusion, can also include the calendering means 13. If present, the calendering means 13 are arranged downstream of the conveying station 12. Then, the calendering means 13 interact with the nonwoven layer on the collection surface 11 to make a nonwoven article.

The configurations of apparatus 100 according to the invention just described enable a new process of making a nonwoven article.

The process is, of course, implemented by apparatus 100.

Thus, the process advantageously includes at least one dispensing stage.

At the dispensing stage, preferably, a layer of filaments of the type chosen from polymeric or cellulose or viscose is dispensed from outlet 10a along the dispensing plane 100a.

Then, the process also advantageously includes a conveying stage.

In the conveying stage, layer filaments are conveyed through the conveying station 12 in an orderly manner onto the collecting surface 11 by orienting the filaments perpendicularly or parallel to the collecting surface 11.

Even more in detail, advantageously, in the conveying stage, the layer is likewise punched so as to form punctures distributed over the layer at which the filaments are interlaced and sealed to each other.

Then, the process also includes a deposition phase. At the deposition stage, preferably, the layer is deposited on the collection surface 11 so that an article is obtained.

If a calender 13 is also present, the process also includes a calendering stage in which the layer is calendered downstream of the conveying station 12 to obtain the article.

Of course, the invention also includes a nonwoven article made by a process as just described.

Plant 1 to make nonwoven fabric, apparatuses 100 to make nonwoven fabric, processes to make a nonwoven fabric article, and nonwoven fabric articles according to the invention achieve important advantages.

In fact, normally spunbond plants of the prior art cannot include spinnerets that are too wide due to the fact that the air, conveyed to the filaments only at the spinneret outlet and laterally, cannot reach the center of the fiber with the consequence that uncooled fibers stick together or do not crystallize with dripping from the spinneret creating serious damage to product quality.

In contrast, plant 1 also allows filaments to be cooled internally in spinneret 4, resulting in more effective and efficient fiber cooling and, most importantly, more uniformly.

Therefore, plant 1 allows for an increase in the number of fibers that are extruded from spinneret 4, thus appreciably increasing the kilograms of filaments produced, compared with current technology;

Also, considering that in spunbond plants of the prior art, cooling air reaches the filaments from the sides and should, theoretically, be able to reach the center of the spinneret, the polymeric fluid outlet drilling layout must always necessarily have holes staggered from each other and spaced far enough apart so that air can reach the center.

In contrast, plant 1 allows filaments to be cooled from above, thus allowing multiple extrusion holes, possibly even multiple spinnerets 4, side by side with different extrusion technology, such as spunbond and melt-blown spinnerets side by side and working together forming a single layer at the same time, on the same plant 1.

Regarding apparatus 100, normally with the technology of the known technique if one intends to produce a nonwoven fabric to be used in the production of diapers, one needs five layers of nonwoven fabric laminated together and afterwards calendered all five together, with the need to have in the machine five different apparatuses equipped with at least three plants with spunbond type spinnerets and plants with meltblown type spinnerets, which, as can be imagined, increase the cost of the product, with more layers of nonwoven fabric being laminated and calendered together anyway resulting in a reduced quality, in mechanical terms, of the final product.

Instead, with the apparatuses 100 according to the invention, it is possible to arrange two or three plants side by side with each other, either in one piece or having separate components, enabling simultaneous work with several spinnerets of different types and thus having, with plants within the same booth 10 the potential of three apparatus heads of the prior art, significantly reducing the costs of investment, maintenance and consumption of electricity and air.

In addition, the simultaneous mixing of different types of filaments, for example, of the SMS type, before exiting booth 10, greatly increases the quality of the article and significantly changes both its technical-mechanical and aesthetic characteristics.

Furthermore, if there are cellulose or viscose dispensing plants, equipped with respective conveyors or editors from which cellulose and or viscose or other material comes out, and polymer filament dispensing plants which at the time they come out meet cellulose and/or viscose, mixing together, apparatus 100 makes it possible to make a single layer of cloth composed partly of polymer fiber and partly of cellulose and or viscose, cotton or other material.

In conclusion, while the known apparatuses allow items with good mechanical strength to be made only in one direction, the apparatuses 100 allow, the conveying station 12 allows filaments to be oriented so that they are deposited on the collection surface 11 not only in one direction but also possibly in a different direction, for example, at an angle to the feed direction of the collection surface 11. This different positioning of the filaments before passing under the calender allows the layer that has been formed to increase its mechanical strength in both directions.

In addition, having a conveying station 12 in which at least one rolling element 12a includes additional needles 121 makes it possible to orient the filaments and, at the same time, perforate the layer formed by them in such a way as to partially seal them together before passing under the calender. This allows for additional mechanical strength on both sides of the article thus made.

In conclusion, apparatuses 100 in which the conveying of the filament layer takes place without depositing on the collecting surface and in which the conveying of different filaments takes place along separate paths so that layers that have already been individually compacted can be calendered without depositing on other layers, allows for the production of better performing and more durable nonwoven articles, whose layers are stronger and the sealing between the different layers is firmer so as to increase the performance of the article, especially when they are used to make diapers or wet wipes.

The invention can be modified to create different versions falling within the scope of the inventive concept defined by the claims.

In this context, all the details can be replaced by equivalent elements and any materials, shapes and dimensions can be used.

## Claims

1. Apparatus (100) for making nonwoven fabric including at least one plant for making nonwoven fabric developing along a main axis (1a) and a main plane (1b) and configured to dispense via an outlet (10a) a plurality of either polymeric or cellulose or viscose filaments along and parallel to one or more dispensing directions (4a) transverse to said main plane (1b) forming from said outlet (10a) a layer of said filaments along or parallel to a dispensing plane (100a) transverse to said main axis (1a) and said main plane (1a); and **characterized by the fact that** it includes
- a conveying station (12) downstream of said outlet (10a) and including at least calendering means (13) spaced from said outlet (10a) by a collection distance of between 0 m and 5 m;
- said conveying station (12) being lacking a belt or mat collection surface placed upstream of said calendering means (13) in such a way that said layer is directly calendered into said calendering means (13) without being previously deposited on a said belt or mat collection surface.

2. Apparatus (100) according to claim 1, wherein said calendering means (13) are spaced from said outlet (10a) by a collection distance of 0.1 m to 3 m.

3. Apparatus (100) according to any preceding claim, wherein said calendering means (13) comprise at least one pair of counter-rotating first rollers (13a) developed parallel to said main plane (1b) and rotating about respective axes of rotation oriented with respect to said main axis (1a) and configured to convey under pressure said layer away from said outlet (10a).

4. Apparatus (100) according to the preceding claim, wherein one or more of said rollers (13a) comprise a first contact surface (130) smooth or including one or more knurls configured to draw said layer from said outlet (10a) when said filaments contact said first contact surface (130) and to release said filaments in an orderly manner when said layer is released from said first contact surface (130).

5. Apparatus (100) according to the preceding claim, wherein said first contact surface (130) comprises a plurality of first needles (131) radially distributed along and around said axis of rotation protruding from said first contact surface (130) and configured to puncture said layer of nonwoven fabric dispensed from said outlet (10a) so as to form distributed punctures on said layer at which said filaments are interlaced and sealed reciprocally.

6. Apparatus (100) according to any preceding claim, wherein said conveying station (12) comprises compacting means (14) located between said outlet (10a) and said calendering means (13) and configured to compress said layer to mutually interlace and seal said filaments of said layer before said layer is conveyed to said calendering means (13).

7. Apparatus (100) according to the preceding claim, wherein said compacting means (14) comprise at least one pair of counter-rotating second rollers (14a) developed parallel to said main plane (1b) and rotating about respective axes of rotation oriented with respect to said main axis (1a) and configured to convey under pressure said layer away from said outlet (10a) towards said calendering means (13).

8. Apparatus (100) according to any preceding claim, wherein said calendering means (13) and said compacting means (14) are placed in sequence along said dispensing plane (100a) in such a manner as to convey said layer along said dispensing plane (100a).

9. Process of making a nonwoven fabric item implemented with an apparatus (100) according to any of the preceding claims, comprising dispensing from said outlet (10a) a layer of filaments of the type chosen from polymeric or cellulose or viscose along said dispensing plane (100a);
and **characterized by the fact that** it includes
- directly conveying said layer to said calendering means (13) from said outlet (10a) without previously depositing said layer on a belt or mat-top collection surface; and
- calendering said layer with said calendering means (13).

10. Nonwoven fabric item made by a process according to claim 9.
